# EUROPÄISCHE PATENTSCHRIFT

(11) **EP 2 911 591 B1**
(45) Veröffentlichungstag und Bekanntmachung des Hinweises auf die Patenterteilung: **17.06.2020**
(21) Anmeldenummer: 13783040.2
(22) Anmeldetag: 24.10.2013
(51) Int. Cl.: A61B 17/02, A61B 17/29

(54) **ATRIUM-RETRAKTOR**
ATRIUM RETRACTOR
RÉTRACTEUR ATRIAL

(30) Priorität: 29.10.2012 DE 102012219727
(43) Veröffentlichungstag der Anmeldung: 02.09.2015
(73) Patentinhaber: Aesculap AG, 78532 Tuttlingen (DE)
(72) Erfinder: SCHABERT, Stefanie, 78554 Aldingen (DE); MORALES, Pedro, 78532 Tuttlingen (DE); BECK, Thomas, 78591 Durchhausen (DE)
(74) Vertreter: Koller, Tobias Kilian
(86) Internationale Anmeldenummer: PCT/EP2013/072241
(87) Internationale Veröffentlichungsnummer: WO 2014/067840

(56) Entgegenhaltungen:
- WO-A1-02/28287
- WO-A2-2007/075903
- US-A1- 2010 286 485

## Beschreibung

Die vorliegende Erfindung betrifft einen Atrium- Retraktor, also eine Vorrichtung zum Anheben der ventralen Wand eines rechten Vorhofs (d.h. Atrium) eines Herzens.

### Stand der Technik

Bei einigen Operationen am Herzen muss dieses in bestimmte Positionen gebracht werden, um einen Zugang des Operateurs zum Operationsfeld zu gewährleisten. Muss beispielsweise ein Zugang zu der Rückseite des Herzens gewährleistet werden, wird das Herz angehoben. Möchte man an der Spitze des Herzens operieren, kann beispielsweise ein Operationstuch unter das Herz geschoben werden, auf dem das Herz dann teilweise aufliegt und so in eine angehobene Stellung gebracht wird. Es gibt aber auch Vorrichtungen, die die Spitze des Herzens mittels Unterdruck ansaugt und dadurch ein Anheben des Herzens ermöglicht.

Darüber hinaus gibt es Vorrichtungen, die einen bestimmten Teil eines Herzens anheben. Bei einer Operation an der Mitralklappe wird die linke Herzkammer aufgeschnitten und die ventrale Wand des linken Vorhofs wird angehoben, um einen Zugang zur Mitralklappe zu schaffen. In der internationalen Patentanmeldung WO 2008/098616 ist ein Atrium-Lift bzw. ein Atrium-Retraktor gezeigt, der über einen gebogenen Schild verfügt, welcher schwenkbar an einem länglichen Schaft vorgesehen ist. Der Winkel zwischen dem Schild und dem Schaft ist über eine Betätigungsvorrichtung am Schaft einstellbar. Auf diese Weise kann der Schild mit der Längsachse des Schafts ausgerichtet werden und so durch eine Inzision in den Brustkorb eines Patienten eingeführt werden. Anschließend wird der Schild durch die Betätigungsrichtung in eine Position gebracht, in der der Schild im Wesentlichen senkrecht zur Längsachse des Schafts ausgerichtet ist. Nun kann der Schild in das geöffnete Atrium des Herzens eingeführt werden und die ventrale Wand angehoben werden. Dieser Stand der Technik hat den Nachteil, dass in der Nähe des Herzens eine recht große Inzision erfolgen muss, um den Schild in den Körper des Patienten einzuführen. Da der Schild zudem gebogen ist, müssen zum Einführen des Retraktors in der Nähe des Herzens zwei benachbarte Rippen mit Hilfe eines weiteren Retraktors aufgespreizt werden.

In der US Patentanmeldung US 2004/0143163 ist zudem ein endoskopischer Atrium-Lift bzw. Atrium-Retraktor gezeigt, bei dem das Schild aus gelenkig miteinander verbundenen Segmenten besteht, die zum Einführen des Retraktors in den Körper des Patienten und zum Herausnehmen zusammen gerollt werden können, sodass sie einen minimalen Durchmesser einnehmen. Diese Vorrichtung hat aber die Nachteile, dass in der Nähe des Herzens eine relativ große Inzision erforderlich ist, um den zusammengerollten Schild dort hindurch einzuführen. Zudem versperrt die Mechanik, welche den Schild aufrollt und zusammen rollt, zumindest teilweise den freien Blick auf die Mitralklappe. Die ventrale Wand des Atriums muss also übermäßig weit angehoben werden, um ein hinreichendes Sichtfeld und einen ausreichenden Zugang frei zu legen.

In der WO 02/28287 A1 ist ein Unterdruck-Niederhalter für Bypass-Operationen offenbart, bei dem ein Gelenk zwischen Schaft und Funktionsende vorgesehen ist, welches mittels eines Innenschafts aktiv geschwenkt werden kann. In der WO 2007/075903 A2 ist ein Atrium-Retraktor gezeigt, bei dem Schaft und Schild lösbar aneinander montierbar sind, sodass der Schild separat vom Schaft in den Brustkorb eines Patienten eingeführt werden kann und anschließend mit dem Schaft verbunden werden kann. In der US2010/0286485 A1 ist ein Weichteil-Retraktor gezeigt, bei dem diverse Valven über ein Zugseil relativ zueinander verlagert werden können.

In der EP2124757 B1, welche den nächstliegenden Stand der Technik bildet, ist ein Atrium-Retraktor gezeigt, bei dem der Schild vom Schaft gelöst werden kann, damit der Schild separat vom Schaft in den Brustkorb des Pateinten eingeführt werden kann und dann mit dem Schaft verbunden werden kann. Zudem verfügt dieser Atrium-Retraktor über zwei Scharniergelenke, von denen eines mittels eines Steuerstabes aktiv geschwenkt werden kann und das andere sich selbsttätig an eine Lageveränderung des Schilds anpasst.

Die Aufgabe der vorliegenden Erfindung ist es daher, einen Atrium- Retraktor zu schaffen, mit dessen Hilfe ein freies Sichtfeld auf die Mitralklappe sowie ein großer Zugang zu dieser geschaffen werden kann, ohne dass es einer großen Inzision in der Nähe des Herzens bedarf.

Eine weitere Aufgabe der vorliegenden Erfindung ist es, einen Atrium- Retraktor zu schaffen, der eine bessere Beweglichkeit des Schilds gegenüber dem Schaft des Retraktors ermöglicht.

Die Aufgabe der vorliegenden Erfindung wird gelöst durch einen Atrium- Retraktor gemäß Anspruch 1. Vorteilhafte Ausgestaltungen und Weiterentwicklungen des erfindungsgemäßen Atrium-Retraktors sind Gegenstand der abhängigen Ansprüche.

Der erfindungsgemäße Atrium-Retraktor weist einen Schild und einen Schaft auf, wobei der Schaft eine Schafthülse, eine Innenstange und einen Betätigungsmechanismus aufweist. Der Betätigungsmechanismus ist derart gestaltet, dass er eine Relativbewegung zwischen der Schafthülse und der Innenstange bewirken kann. Eine Gelenkvorrichtung ist zudem entweder dem Schild oder dem Schaft fest verbunden und ist mit dem anderen von dem Schild oder dem Schaft lösbar verbindbar. Dies bedeutet, dass die Gelenkverbindung an einem Bauteil fest montiert und an dem anderen Bauteil lösbar montiert ist. Hierbei ist es aber nicht entscheidend, dass die feste Verbindung unlösbar ist, wie dies beispielsweise bei einer stofflichen Verbindung durch Schweißen o.ä. der Fall ist, sondern dass diese Verbindung so fest ist, dass sie nicht versehentlich gelöst wird, wenn die lösbare Verbindung zum anderen Bauteil gelöst werden soll. Sind beide Verbindungen beispielsweise Schraubverbindungen, dann solle die feste Verbindung beispielsweise mit einer Kontermutter gesichert sein, damit sich diese Verbindung nicht löst, wenn die lösbare Verbindung gelöst wird. Die Betätigungsvorrichtung ist zudem daran angepasst, die Gelenkverbindung über die Relativbewegung zwischen der Schafthülse und der Innenstange wahlweise zu blockieren und freizugeben.

Darüber hinaus ist die Gelenkverbindung fest mit dem Schild verbunden und ist lösbar mit dem Schaft verbindbar. Dies bedeutet, dass die Gelenkvorrichtung mit dem Schild zusammen in den Brustkorb eingeführt und aus diesem wieder entfernt wird. Auf diese Weise kann die frontale Inzision (d.h. die Inzision verläuft in der Frontalebene des Patienten, also auf der Vorderseite des Brustkorbs) minimiert werden. Die Gelenkverbindung weist bei dieser Ausführungsform ein erstes Bauteil, welches starr an dem Schild angebracht ist, und ein zweites Bauteil auf, welches gegenüber dem Schild bewegbar und insbesondere schwenkbar ist. Die Gelenkverbindung wird dementsprechend durch ein Kugelgelenk gebildet. Mit einem Kugelgelenk ist eine Einstellung um drei Achsen möglich, der Schild kann also im Vergleich zu einem Sattelgelenk auch noch gegenüber dem Schaft um die Schaftachse gedreht werden.

Zudem ist die Gelenkverbindung eine Kugelgelenkverbindung, die eine Gelenkkugel und eine Kugelhülse aufweist. Mit einer solchen Kugelgelenkverbindung wird ein besonders freies Einstellen der Lage des Schilds zu dem Schaft ermöglicht. Die Gelenkkugel ist dabei starr an dem Schild vorgesehen und die Kugelhülse weist ein Gewinde auf. Die Schafthülse weist ebenfalls ein Gewinde auf und mit dem Gewinde der Kugelhülse verschraubbar. Auf diese Weise wird die lösbare Verbindung zwischen dem Schaft und der Gelenkverbindung bzw. dem Schild hergestellt. Die Innenstange ist darüber hinaus eine Druckstange, die daran angepasst ist, im betätigten Zustand der Betätigungsvorrichtung einen Druck auf die Gelenkkugel auszuüben, um die Gelenkkugel gegen einen Sitz zu drücken, der an der Gelenkhülse ausgebildet ist, um die Gelenkverbindung zu blockieren.
Hierbei ist es zunächst unerheblich, ob die Gelenkverbindung im betätigten Zustand der Betätigungsvorrichtung blockiert oder freigegeben ist und im nicht betätigten Zustand der Betätigungsvorrichtung dementsprechend freigegeben oder blockiert ist. Mit einem solchen Atrium-Retraktor ist es möglich, die Lage des Schilds gegenüber dem Schaft zu verändern, wenn die Gelenkverbindung freigegeben ist, und die Lage zwischen Schaft und Schild zu sichern, wenn die Gelenkvorrichtung blockiert ist. Der Schild kann dementsprechend viele Stellungen gegenüber dem Schaft einnehmen und halten. Um die Lage des Schilds gegenüber dem Schaft präzise einzustellen, wird der Schild beispielsweise durch einen gewöhnlichen Nadelhalter gegriffen und gegenüber dem Schaft, welcher zumeist entweder durch eine Hand eines Operateurs oder eines Hilfspersonals gegriffen ist oder an einer Haltevorrichtung befestigt ist, bewegt, bis die gewünschte Lage erreicht ist. Anschließend wird die Gelenkvorrichtung blockiert, sodass die Stellung des Schilds gegenüber dem Schaft fixiert ist.

Bei einer Operation an der Mitralklappe wird zwischen zwei benachbarten rechten Rippen des Patienten eine Inzision vorgenommen - meist zwischen der fünften und sechsten rechten Rippe - und diese Inzision und die zugehörigen Rippen mit einem herkömmlichen Retraktor aufgespreizt. Von diesem Zugang aus hat der Operateur Sicht auf das spätere Operationsfeld am Herzen und an der Mitralklappe und führt die Operation durch. Anschließend wird die linke Herzkammer aufgeschnitten. Um die ventrale Wand der Herzkammer anheben zu können, wird eine weitere kleine Inzision knapp neben dem Herzen von vorne in den Brustkorb vorgenommen. Diese Inzision kann sehr klein sein und muss nicht durch einen Retraktor aufgespreizt werden, da nur das distale Ende des Schafts des Atrium- Retraktors dort hindurch geführt werden muss. Der Schild wird durch die größere Öffnung auf der rechten Seite des Brustkorbes eingeführt und mit Hilfe eines Nadelhalters oder eines vergleichbaren Instruments an das Herz herangeführt. Dann wird das distale Ende des Schafts durch die kleine frontale Inzision eingeführt und mit dem Schild gekoppelt. Anschließend stellt der Operateur die gewünschte Stellung zwischen dem Schild und dem Schaft ein und blockiert die Gelenkverbindung. Zum Einstellen der gewünschten Stellung des Schilds und für eventuelle Korrekturen der Stellung während der Operation, wird der Schild vorzugsweise durch ein Instrument wie beispielsweise einen Nadelhalter festgehalten. Nun kann er den Schild des Atrium- Retraktors zwischen die ventrale und die dorsale Wand der Herzkammer schieben und durch die ventrale Wand der Herzkammer angeben, indem er den Schaft ein Stück weit aus dem Brustkorb des Patienten heraus zieht.

Wenn der Atrium- Retraktor nicht mehr benötigt wird, kann der Schild wieder durch ein Instrument gegriffen werden und die lösbare Verbindung zwischen der Gelenkverbindung und dem Schild bzw. dem Schaft wird gelöst. Der Schaft wird anschließend ggf. mitsamt der Gelenkverbindung nach vorn aus dem Brustkorb heraus gezogen und der Schild wird (auch ggf. mitsamt der Gelenkverbindung) seitlich durch die Öffnung im rechten Brustkorb entfernt.

Gemäß einer vorteilhaften Ausführungsform der vorliegenden Erfindung ist die Betätigungsvorrichtung daran angepasst, die Relativbewegung zwischen der Schafthülse und der Innenstange wahlweise mittels Reibschluss zu blockieren. Ein Reibschluss als Blockiermechanismus ist insofern vorteilhaft, da er unabhängig von der Stellung des Schilds zu dem Schaft realisierbar ist und eine stufenlose Einstellung ermöglicht. Bei formschlüssigen Blockiermechanismen ist eine stufenlose Einstellung nicht oder nur sehr schwer möglich, dennoch sind formschlüssige Blockiermechanismen realisierbar, insbesondere wenn es eine bestimmte Anzahl von bevorzugten Stellungen des Schilds zum Schaft gibt.

Gemäß noch einer weiteren vorteilhaften Ausführungsform der vorliegenden Erfindung ist in der Kugelhülse eine Abstützung vorgesehen, an der sich ein elastisches Element abstützt, welches zwischen Kugelhülse und Gelenkkugel angeordnet ist, um die Gelenkkugel im freigegebenen Zustand der Gelenkverbindung mit einer vorbestimmten Kraft gegen den Sitz zu drücken. Auf diese Weise wird ein unkontrolliertes Bewegen der Kugelhülse gegenüber dem Schild verhindert. Dies bringt den Vorteil mit sich, dass das distale Ende des Schafts einfacher in die Öffnung der Gelenkhülse eingeführt werden kann, da diese dem Schaftende nicht so einfach ausweicht. Außerdem ist es vorteilhaft, wenn sich die Gelenkhülse nicht kraftfrei zu dem Schild um ihre Achse drehen kann, da in diesem Fall ein Einschrauben des Schafts bzw. der Schafthülse in die Gelenkhülse nur möglich wäre, wenn die Gelenkhülse festgehalten werden würde. Dies müsste mit einem zusätzlichen Instrument erfolgen, was aufgrund der sehr begrenzten Platzverhältnisse schwierig und kompliziert ist. Das elastische Element ist dabei vorzugsweise eine Feder und insbesondere eine Blattfeder oder Tellerfeder. Vor allem mit einer Tellerfeder kann die Gelenkhülse klein dimensioniert werden und gleichzeitig kann eine ausreichende Kraft auf die Kugel ausgeübt werden, um eine Reibung zwischen Gelenkkugel und Gelenkhülse zu erzielen, die ein Einschrauben der Schafthülse in die Gelenkhülse ermöglicht.

Gemäß einer vorteilhaften Ausführungsform der vorliegenden Erfindung weist die Gelenkhülse zwei Teile auf, die lösbar oder unlösbar miteinander verbunden sind. Der Sitz und die Abstützung sind vorzugsweise an verschiedenen Teilen der Gelenkhülse vorgesehen. Auf diese Weise können die einzelnen Teile leicht hergestellt werden und auch der Zusammenbau der Gelenkvorrichtung ist auf diese Weise sehr einfach.

Gemäß einer besonders vorteilhaften Ausführungsform der vorliegenden Erfindung ist der Schild gebogen und/oder an seinem distalen Ende einen lateral verlaufenden Vorsprung versehen, der bei Verwendung des Atrium-Retraktors von dem Schild nach ventral vorsteht. Durch die laterale Biegung des Schilds ist er besonders gut an die anatomischen Verhältnisse der ventralen Herzkammerwand angepasst. Der lateral verlaufende Vorsprung dient dazu, ein versehentliches Abrutschen der Herzkammerwand von dem Schild zu verhindern.

Gemäß einer weiteren besonders vorteilhaften Ausführungsform der vorliegenden Erfindung weist der Schild einen Greifbereich auf der daran angepasst ist, von einem Instrument gegriffen zu werden. Wie zuvor beschrieben, wird der Schild über einen relativ weiten Bereich seitlich in den Brustkorb eingeschoben. Um auch diese Inzision möglichst klein zu halten und um den Schild kontrolliert durch den Brustkorb zu transportieren, wird er zumeist mit einem Instrument wie beispielsweise einem Nadelhalter gegriffen und geführt. Der Greifbereich ist dabei vorzugsweise so ausgebildet, dass ein Schwenken, also ein seitliches Verdrehen, des Schilds gegenüber dem Instrument verhindert ist. Dazu ist der Greifbereich zum Beispiel als ein Bereich am proximalen Ende des Schilds ausgebildet, der gegenüber den übrigen Schild eine verringerte Dicke aufweist. Möchte sich nun der Schild gegenüber dem Instrument seitlich verdrehen, mit dem er eingeführt wird, so stoßen die seitlichen Wände des Greifbereichs gegen die Backen des Instruments. Auf diese Weise ist ein Verdrehen des Schilds gegenüber dem Instrument verhindert oder zumindest begrenzt. Alternativ dazu kann der Greifbereich aber auch durch seitliche Begrenzungen in Form von Vorsprüngen oder ähnlichem definiert sein.

Gemäß einer anderen vorteilhaften Ausführungsform der vorliegenden Erfindung weist der Atrium- Retraktor wenigstens einen Erweiterungsschild auf, welcher lösbar an dem Schild des Retraktors anbringbar ist. Dabei weist der Schild im Bereich wenigstens eines lateralen Randes eine Nut auf, in die eine Feder des Erweiterungsschilds einschiebbar ist, sodass der Schild und der Erweiterungsschild im Wesentlichen eine gemeinsame Schildfläche bilden. Das Einschieben des Erweiterungsschilds in die Nut an dem Schild erfolgt vorzugsweise von dem hinteren Ende des Schilds aus, also entgegengesetzt zu dem Ende, an dem lateral verlaufenden Vorsprung in einigen Fällen angeordnet ist. Zudem ist die Nut vorzugsweise zum vorderen Ende des Schildes hin verengt oder geschlossen, sodass der Erweiterungsschild nicht nach vorn aus der Nut des Schildes heraus geschoben werden kann.

Gemäß einer besonders vorteilhaften Ausführungsform der vorliegenden Erfindung ist die wenigstens eine Nut in der lateralen Seitenfläche des Schildes ausgebildet. Auf diese Weise kann ein besonders sanfter Übergang von der Außenfläche bzw. Oberfläche des Schildes zu der Außenfläche bzw. Oberfläche des Erweiterungsschildes geschaffen werden. Zudem wird auf diese Weise das Blickfeld auf das Operationsgebiet nicht beschränkt, wie es zum Beispiel der Fall wäre, wenn die Nut auf der Unterseite des Schildes angeordnet wäre.

Gemäß noch einer anderen vorteilhaften Ausführungsform der vorliegenden Erfindung ist der Erweiterungsschild daran angepasst, in lateraler Richtung plastisch biegbar zu sein. Dies wird bewerkstelligt, indem der Erweiterungsschild aus einem zumindest teilweise plastischen Material ausgebildet wird, beispielsweise aus einem Metallblech. Die Dicke und das Material des Metallblechs sind so gewählt, dass der Erweiterungsschild durch Muskelkraft des Operateurs gebogen werden kann, ggf. mit Hilfe von Zangen oder ähnlichem. Der Schild weist vorzugsweise mindestens eine Aussparung auf, die sich von seinem hinteren Rand zu dem vorderen Bereich des Erweiterungsschilds erstreckt. In diesem Fall ist der erweiterungsschild im Bereich der Aussparungen besonders leicht zu biegen, gleichzeitig wird aber die Abschirmwirkung des Erweiterungsschildes nur sehr wenig beeinträchtigt. Die ventrale Wand der Herzkammer ist ein Muskelgewebe, welches eine gewisse Festigkeit aufweist. Es ist also nicht so, dass das Gewebe im Bereich der Aussparungen weit in den abgeschirmten Raum innerhalb bzw. unterhalb des Erweiterungsschildes eintritt.

Gemäß einer vorteilhaften Ausführungsform der vorliegenden Erfindung ist die Betätigungsvorrichtung ein Exzenterhebel mit einer Exzenterfläche, wobei der Exzenterhebel gelenkig an der Schafthülse befestigt ist und mit seiner Exzenterfläche an dem proximalen Ende der Druckstange anliegt. Alternativ dazu kann auch eine Drehkulisse als Betätigungsvorrichtung vorgesehen sein, die bei einer Drehung um die Längsachse des Schafts eine Verschiebung der Schafthülse zu der Innenstange bewirkt. Ein Exzenterhebel ist aber dahingehend vorteilhaft, dass er einen schnellen Übergang von der freigegebenen Position zur blockierten Position der Gelenkvorrichtung ermöglicht. Außerdem eignet sich ein Exzenterhebel auch für eine Betätigung mit nur einer Hand und für das Aufbringen von großen Kräften. Wird zudem der Schaft über ein Gewinde am Gelenkabschnitt montiert, kann die durch den Exzenterhebel maximal aufbringbare Kraft dadurch eingestellt werden, dass der Schaft unterschiedlich weit in das Gewinde eingeschraubt wird. Auf diese Weise kann auch ein übermäßiger verschleiß verhindert werden, da keine größere Klemmkraft aufgebracht werden muss, nur um den Exzenterhebel zu schließen. In diesem Fall kann der Schaft einfach ein wenig aus dem Gewinde heraus gedreht werden, wodurch sich die in der Schließstellung des Exzenterhebels aufgebrachte Kraft verringert.

Gemäß einer weiteren vorteilhaften Ausführungsform der vorliegenden Erfindung ist der Exzenterhebel über eine Griffhülse an der Schafthülse befestigt. Die Griffhülse weist dabei eine Durchgangsbohrung und die Druckstange ein Langloch auf. Das Langloch kann im Prinzip auch ein Rundloch sein, dessen Durchmesser größer als der des Stiftes ist, welcher sich durch die Durchgangsbohrung in der Griffhülse und das Langloch bzw. das Rundloch in der Druckstange erstreckt. Wichtig ist lediglich, dass ein gewisses Bewegungsvermögen zwischen dem Stift und dem Langloch bzw. Rundloch vorhanden ist, sodass sich Griffhülse (und damit Schafthülse) und Druckstange zueinander in axialer Richtung bewegen können. Auf diese Weise wird eine maximale Bewegung der Druckstange gegenüber der Schafthülse insbesondere im nicht betätigten Zustand der Betätigungsvorrichtung begrenzt. So kann zum Beispiel verhindert werden, dass sich ein Betätigungsarm des Exzenterhebels auf die gegenüberliegende Seite des Schafts bewegt und unter Umständen fehlerhaft betätigt wird.

Gemäß noch einer weiteren vorteilhaften Ausführungsform der vorliegenden Erfindung weist der Exzenterhebel einen Betätigungsarm auf, der im betätigten Zustand der Betätigungsvorrichtung im Wesentlichen parallel zu der Schafthülse verläuft und vorzugsweise im Wesentlichen an der Schafthülse anliegt. Dies ist insbesondere für eine Einhandbetätigung vorteilhaft, da der Bewegungsweg des Betätigungsarms in der Nähe des Schafts erfolgen kann, der für eine Betätigung ebenfalls ergriffen werden muss. Außerdem verringert die Anordnung des Betätigungsarms in der Nähe des Schaftes die Gefahr, dass sich jemand oder etwas versehentlich an dem Betätigungsarm einfädelt und so den Atrium-Retraktor ungewollt verschiebt, verdreht oder sogar die Gelenkvorrichtung löst.

Gemäß einer vorteilhaften Ausführungsform der vorliegenden Erfindung verfügt die Griffhülse über einen Vorsprung, der in eine Nut eingreift, welche in dem Exzenterhebel vorgesehen ist und sich kreisbogenförmig um das Drehzentrum des Exzenterhebels erstreckt, um die Drehbewegung des Exzenterhebels zu der Schafthülse zu begrenzen. Auch dieses Merkmal dient dazu, die Betätigung und die Montage des Atrium-Retraktors sicherer zu machen, indem der Betätigungsgriff nicht auf die gegenüberliegende Seite des Schafts schwenken kann und dann möglicherweise falsch betätigt wird. Vorzugsweise ist dieser Vorsprung durch eine federbelastete Kugel gebildet, die in der Nut läuft, und die Nut verfügt an der Stelle, an der sich die Kugel in der Nut befindet, wenn der Exzenterhebel im Wesentlichen vollständig geöffnet ist, über eine Vertiefung. Auf diese Weise wird der Exzenterhebel durch die federbelastete Kugel in der Öffnungsstellung gehalten, bis durch den Nutzer eine Kraft auf den Exzenterhebel aufgebracht wird, welche die Federkraft der Feder überwindet, welche die Kugel zu der Nut hin drängt.

Gemäß einer anderen vorteilhaften Ausführungsform der vorliegenden Erfindung weist der Atrium-Retraktor einen Stützfuß oder einen Stützteller, der an dem Schaft angebracht oder anbringbar ist und daran angepasst ist, sich an einem Brustbereich des Patienten abzustützen. Auf diese Weise muss der Atrium- Retraktor nicht durch Hilfspersonal gehalten werden. Außerdem kann er präziser positioniert werden. Noch vorteilhafter ist es allerdings, wenn der Atrium- Retraktor an einer Halteeinrichtung montierbar ist, beispielsweise an einem anderen Retraktor oder an dem Operationstisch. Dabei ist es auch möglich, dass der Atrium-Retraktor mittelbar an dem Operationstisch montierbar ist.

Weitere Vorteile und Merkmale der Erfindung sind dem Fachmann aus den beigefügten Figuren und der detaillierten Beschreibung der Ausführungsbeispiele ersichtlich.
- Fg. 1: zeigt eine isometrische Ansicht eines Atrium-Retraktors gemäß einem Ausführungsbeispiel der vorliegenden Erfindung;

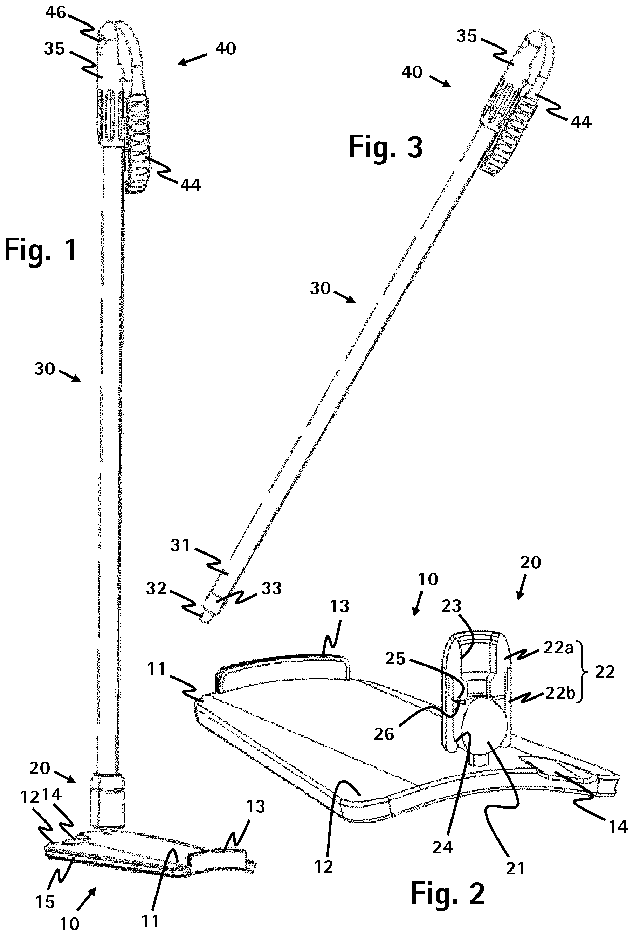

- Fig. 2: zeigt eine isometrische Ansicht des Schilds des Atrium- Retraktors aus Fig. 1 mit teilweise freigeschnittener Gelenkvorrichtung;
- Fig. 3: zeigt eine isometrische Ansicht eines Schafts des Atrium-Retraktors aus Fig. 1;
- Fig. 4: zeigt eine perspektivische Ansicht eines Griffabschnitts des Schafts aus Fig. 1 mit teilweise freigeschnittener Griffhülse;

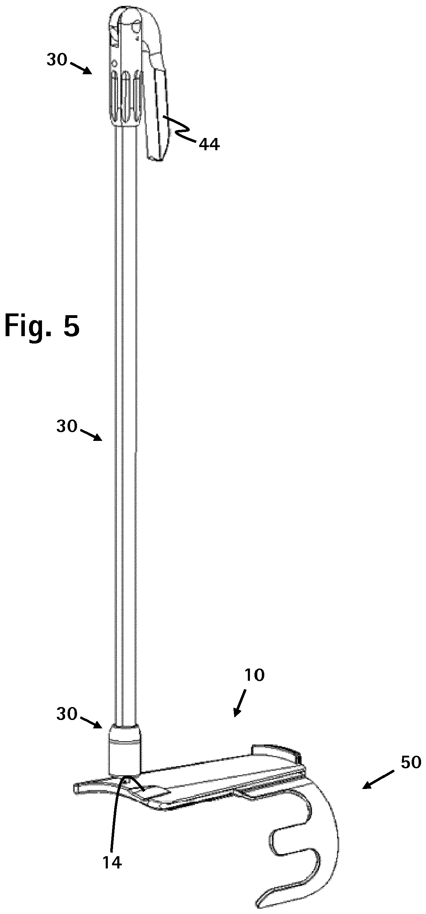

- Fig. 5: zeigt eine isometrische Ansicht des Atrium- Retraktors aus Fig. 1 mit einem Erweiterungsschild;
- Fig. 6: zeigt eine isometrische Ansicht des Schilds und Erweiterungsschilds aus Fig. 5; und
- Fig. 7: zeigt einen Erweiterungsschild entsprechend Fig. 5, wobei Fig. 7A den Schild im Ausgangszustand zeigt und Fig. 7B den Schild in einem passend zurecht gebogenen Zustand zeigt.

Ein Ausführungsbeispiel der vorliegenden Erfindung ist im Folgenden unter Bezugnahme auf die Figuren im Detail beschrieben.

Der Atrium-Retraktor gemäß diesem Ausführungsbeispiel weist einen Schild 10 und einen Schaft 30 auf, welcher eine Schafthülse 31, eine Druckstange 32 und einen Betätigungsmechanismus 40 aufweist. Der Betätigungsmechanismus 40 ist gestaltet, um eine Relativbewegung zwischen der Schafthülse 31 und der Innenstange 32 zu bewirken. Eine Kugelgelenkverbindung 21, 22, die eine Gelenkkugel 21 und eine Kugelhülse 22 aufweist, ist fest mit dem Schild 10 verbunden. Genauer gesagt ist die Gelenkkugel 21 fest mit dem Schild 10 verbunden und die Gelenkhülse 22 verfügt über ein Gewinde 23, in welches ein Außengewinde 33 einschraubbar ist, welches an dem distalen Ende der Schafthülse 31 ausgebildet ist.

In der Gelenkhülse 22, die aus zwei Bauteilen 22a, 22b besteht, ist an dem Bauteil 22a eine Abstützung 25 vorgesehen, an der sich eine Tellerfeder 26 abstützt. Die Tellerfeder 26 stützt sich auf ihrer gegenüberliegenden Seite an der Gelenkkugel 21 ab und drückt die Gelenkkugel 21 im freigegebenen Zustand der Gelenkverbindung 20 mit einer vorbestimmten Kraft gegen den Sitz 24, der an dem anderen Bauteil 22a der Gelenkhülse 22 vorgesehen ist. Auf diese Weise wird ein unkontrolliertes Bewegen der Gelenkhülse 22 gegenüber dem Schild 10 und somit eine ungewollte Bewegung des Schilds 10 gegenüber dem Schaft 30 verhindert.

Der Schild 10 des Atrium-Retraktors ist gebogen und weist an seinem vorderen Ende 11 einen lateral verlaufenden Vorsprung 13 auf, der bei Verwendung des Atrium-Retraktors von dem Schild 10 nach ventral vorsteht, also zu der Innenseite der ventralen Wand der Herzkammer. Zudem weist der Schild 10 einen Greifbereich 14 an seinem hinteren Ende 12 auf, der daran angepasst ist, von einem Instrument gegriffen zu werden. Der Greifbereich 14 ist hier als eine Stelle ausgebildet, an der der Schild 10 eine geringere Dicke als an anderen Stellen aufweist. Dadurch werden an dem Greifbereich Seitenwände ausgebildet, die in Anlage an die Branchen des Instruments gelangen können, welches den Schild greift, und dadurch eine seitliche Verdrehung des Schilds gegenüber dem Instrument verhindern.

Darüber hinaus weist der Atrium-Retraktor dieses Ausführungsbeispiels einen Erweiterungsschild 50 auf, der lösbar an dem Schild 10 des Atrium- Retraktors anbringbar ist. Der Schild 10 weist in seiner rechten lateralen Seitenfläche eine Nut 15 auf, in die eine Feder 51 des Erweiterungsschilds 50 einschiebbar ist. Der Schild 10 und der Erweiterungsschild 50 bilden so im Wesentlichen eine gemeinsame Schildfläche aus. Der Erweiterungsschild 50 ist in seiner Ausgangsstellung eben und ist daran angepasst, in lateraler Richtung plastisch gebogen zu werden, beispielsweise durch den Operateur oder sein Hilfspersonal. Der Erweiterungsschild kann aber auch mit einer gewissen Biegung ausgeliefert werden, sodass durch den Operateur nur noch geringfügige Anpassungen an die individuellen Gegebenheiten im Operationsgebiet erforderlich sind. Der Erweiterungsschild 50 weist zwei Aussparungen 52 auf, die sich von dem hinteren Rand zu dem vorderen Bereich 54 des Erweiterungsschilds 50 erstrecken. Die Dadurch ausgebildeten Finger 53 stehen von dem vorderen Bereich 54 nach hinten zu dem hinteren Ende 12 des Schilds hin vor. Die Finger 53 sind in diesem Fall kürzer als der Schild 10, können aber auch dieselbe Länge wie der Schild 10 aufweisen. Die Nut 15 in dem Schild 10 ist an ihrem vorderen Ende geschlossen, sodass der Erweiterungsschild 50 nicht nach vorne aus der Nut 15 heraus geschoben werden kann.

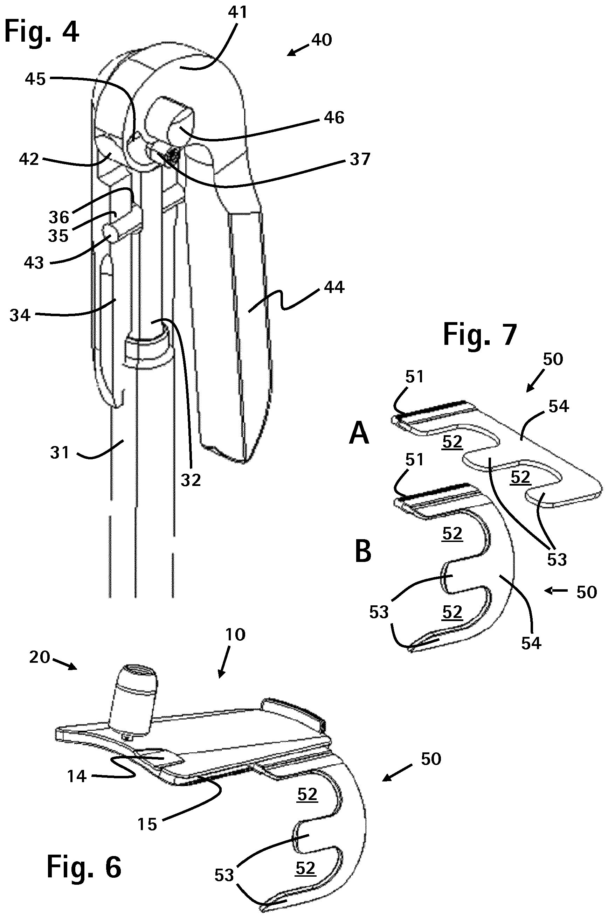

Die Betätigungsvorrichtung 40 ist durch einen Exzenterhebel 41 mit einer Exzenterfläche 42 gebildet. Der Exzenterhebel 41 ist gelenkig an der Griffhülse 35 befestigt, welche wiederum an der Schafthülse 31 befestigt ist. Die Exzenterfläche 42 liegt an dem proximalen Ende der Druckstange 32 an und drückt die Druckstange 32 im Betätigten Zustand, also der in dem in der Fig. 4 gezeigten Stellung, die Druckstange auf die Gelenkkugel 21, sodass der Schild 10 durch Reibschluss zwischen der Druckstange 32 und der Gelenkkugel 21, aber vor allem durch den Reibschluss zwischen Gelenkkugel 21 und Sitz 24 fest gegenüber dem Schaft gehalten wird.

Die Griffhülse 34 weist eine Durchgangsbohrung 35 auf und die Druckstange 32 weist ein Langloch 36 auf. Ein Stift 43 erstreckt sich durch die Durchgangsbohrung 35 in der Griffhülse 34 und das Langloch 36 in der Druckstange 32 und begrenzt so eine maximale Bewegung der Druckstange 32 gegenüber der Schafthülse 31. Der Exzenterhebel 41 weist einen Betätigungsarm 44 auf, der im betätigten Zustand der Betätigungsvorrichtung 40 im Wesentlichen parallel zu der Schafthülse 31 verläuft. Der Betätigungshebel liegt nicht an der Schafthülse 31 an, lässt aber nur einen geringen Spalt zwischen sich und der Schafthülse 31. Die Griffhülse 35 verfügt über einen Vorsprung 37, der in eine Nut 45 eingreift, welche in dem Exzenterhebel 41 vorgesehen ist und sich kreisbogenförmig um das Drehzentrum 46 des Exzenterhebels 41 erstreckt. Bei diesem Ausführungsbeispiel besteht der Vorsprung 37 aus einer Feder und einer Kugel, wobei die Feder die Kugel zu der Nut 45 hin vorspannt. Die Kugel kann sich nur axial zu dem Loch bewegen, in dem die Feder angeordnet ist. Schlägt die Kugel 37 an den seitlichen Stirnwänden der Nut 45 an, begrenzt diese Anlage die Drehbewegung des Exzenterhebels 41 zu der Schafthülse 31. Die Nut 45 weist zudem an der Stelle, an der sich die Kugel befindet, wenn der Exzenterhebel 41 im Wesentlichen vollständig geöffnet ist, eine Vertiefung auf. Die Kugel wird durch die Feder in die Vertiefung gedrängt, wenn der Exzenterhebel 41 im Wesentlichen vollständig geöffnet ist, und hält den Exzenterhebel 41 dadurch in dieser Stellung. Nun kann der Operateur die Lage des Schafts zu dem Schild einstellen, ohne den Exzenterhebel 41 in der geöffneten Stellung halten zu müssen. Um den Exzenterhebel 41 zu schließen, muss zunächst die Kraft überwunden werden, mit der die Feder die Kugel in die Nut 45 drängt.

Der vorliegende Atrium-Retraktor ist zudem an einer Haltevorrichtung befestigbar. Hierzu kann ein nicht gezeigtes, herkömmliches Befestigungsmittel verwendet werden.

Die vorstehend beschriebene Erfindung kann in vielfältiger Weise ausgeführt und modifiziert werden und die konkret beschriebene Ausführungsform ist nicht als Beschränkung des Schutzumfangs zu verstehen, wie er in den Ansprüchen definiert ist.

## Patentansprüche

1. Atrium-Retraktor mit
einem Schild (10) und
einem Schaft (30), der eine Schafthülse (31), eine Druckstange (32) und einen Betätigungsmechanismus (40) aufweist, wobei der Betätigungsmechanismus (40) gestaltet ist, um eine axiale Relativbewegung zwischen der Schafthülse (31) und der Druckstange (32) zu bewirken,
einer Gelenkvorrichtung (20), die mit dem Schild (10) fest verbunden ist und mit dem Schaft (30) lösbar verbindbar ist, wobei der Betätigungsmechanismus (40) daran angepasst ist, die Gelenkverbindung (20) über die axiale Relativbewegung zwischen der Schafthülse (31) und der Druckstange (32) wahlweise zu blockieren und freizugeben, wobei
die Gelenkverbindung (20) ein erstes Bauteil (21), welches starr an dem Schild (10) angebracht ist, und ein zweites Bauteil (22) aufweist, welches gegenüber dem Schild (10) bewegbar und insbesondere schwenkbar ist,
die Gelenkverbindung (20) eine Kugelgelenkverbindung (21, 22) ist, die eine Gelenkkugel (21) und eine Kugelhülse (22) aufweist, wobei
die Gelenkkugel (21) starr an dem Schild (10) vorgesehen ist und die Kugelhülse (22) ein Gewinde (23) aufweist,
die Schafthülse (30) ein Gewinde (33) aufweist und mit dem Gewinde (23) der Kugelhülse (22) verschraubbar ist, und wobei
die Druckstange (32) daran angepasst ist, im betätigten Zustand der Betätigungsvorrichtung (40) einen Druck auf die Gelenkkugel (21) auszuüben, um die Gelenkkugel (21) gegen einen Sitz (24) zu drücken, der an der Gelenkhülse (22) ausgebildet ist, um die Gelenkverbindung (20) zu blockieren.

2. Atrium-Retraktor nach Anspruch 1, wobei
die Betätigungsvorrichtung daran angepasst ist, die Relativbewegung zwischen der Schafthülse (31) und der Druckstange (32) wahlweise mittels Reibschluss zu blockieren.

3. Atrium-Retraktor nach Anspruch 1 oder 2, wobei
in der Gelenkhülse (22) eine Abstützung (25) vorgesehen ist, an der sich ein elastisches Element (26) abstützt, welches sich auf der entgegengesetzten Seite an der Gelenkkugel (21) abstützt, um die Gelenkkugel (21) im freigegebenen Zustand der Gelenkverbindung (20) mit einer vorbestimmten Kraft gegen den Sitz (24) zu drücken, um ein unkontrolliertes Bewegen der Gelenkhülse (22) gegenüber dem Schild (10) zu verhindern, wobei das elastische Element (26) vorzugsweise eine Feder und insbesondere eine Blattfeder oder Tellerfeder ist,

4. Atrium-Retraktor nach einem der vorangehenden Ansprüche, wobei
die Gelenkhülse (22) zwei Teile (22a, 22b) aufweist, die lösbar oder unlösbar miteinander verbunden sind, wobei der Sitz (24) und die Abstützung (25) vorzugsweise an verschiedenen Teilen (22a, 22b) der Gelenkhülse (22) vorgesehen sind.

5. Atrium-Retraktor nach einem der vorangehenden Ansprüche, wobei
der Schild (10) gebogen ist und/oder an seinem vorderen Ende (11) einen lateral verlaufenden Vorsprung (13) aufweist, der bei Verwendung des Atrium-Retraktors von dem Schild (10) nach ventral vorsteht.

6. Atrium-Retraktor nach einem der vorangehenden Ansprüche, wobei
der Schild (10) einen Greifbereich (14) aufweist der daran angepasst ist, von einem Instrument gegriffen zu werden, wobei der Greifbereich (14) vorzugsweise so ausgebildet ist, dass ein Schwenken des Schilds (10) gegenüber dem Instrument verhindert ist.

7. Atrium-Retraktor nach einem der vorangehenden Ansprüche mit
wenigstens einem Erweiterungsschild (50), welcher lösbar an dem Schild (10) des Atrium- Retraktors anbringbar ist, wobei
der Schild (10) im Bereich wenigstens eines lateralen Randes eine Nut (15) aufweist, in die eine Feder (51) des Erweiterungsschilds (50) einschiebbar ist, sodass der Schild (10) und der Erweiterungsschild (50) im Wesentlichen eine gemeinsame Schildfläche bilden.

8. Atrium-Retraktor nach Anspruch 7, wobei
die wenigstens eine Nut (15) in der lateralen Seitenfläche des Schildes (10) ausgebildet ist.

9. Atrium-Retraktor nach einem der Ansprüche 7 oder 8, wobei
der Erweiterungsschild (50) daran angepasst ist, in lateraler Richtung plastisch biegbar zu sein, wobei der Erweiterungsschild (50) vorzugsweise mindestens eine Aussparung (52) aufweist, die sich von dem hinteren Rand zu dem vorderen Bereich (54) des Erweiterungsschilds (50) erstreckt.

10. Atrium-Retraktor nach einem der vorangehenden Ansprüche, wobei
die Betätigungsvorrichtung (40) ein Exzenterhebel (41) mit einer Exzenterfläche (42) ist, wobei der Exzenterhebel (41) gelenkig an der Schafthülse (31) befestigt ist und mit seiner Exzenterfläche (42) an dem proximalen Ende der Druckstange (32) anliegt.

11. Atrium-Retraktor nach Anspruch 10, wobei
der Exzenterhebel (41) über eine Griff hülse (34) an der Schafthülse (31) befestigt ist,
die Griffhülse (34) eine Durchgangsbohrung (35) aufweist, und
die Druckstange (32) ein Langloch (36) aufweist, wobei sich ein Stift (43) durch die Durchgangsbohrung (35) in der Griffhülse (34) und das Langloch (36) in der Druckstange (32) erstreckt und auf diese Weise eine maximale Bewegung der Druckstange (32) gegenüber der Schafthülse (31) insbesondere im nicht betätigten Zustand der Betätigungsvorrichtung (40) zu begrenzen.

12. Atrium-Retraktor nach einem der Ansprüche 10 oder 11, wobei
der Exzenterhebel (41) einen Betätigungsarm (44) aufweist, der im betätigten Zustand der Betätigungsvorrichtung (40) im Wesentlichen parallel zu der Schafthülse (31) verläuft und vorzugsweise im Wesentlichen an der Schafthülse (31) anliegt.

13. Atrium-Retraktor nach einem der Ansprüche 10 bis 12, wobei
die Griffhülse (35) über einen Vorsprung (37) verfügt, der in eine Nut (45) eingreift, welche in dem Exzenterhebel (41) vorgesehen ist und sich kreisbogenförmig um das Drehzentrum (46) des Exzenterhebels (41) erstreckt, um die Drehbewegung des Exzenterhebels (41) zu der Schafthülse (31) zu begrenzen.

## Claims

1. Atrium retractor with
a plate (10) and
a shaft (30) which has a shaft sleeve (31), a thrust rod (32) and an actuating mechanism (40), wherein the actuating mechanism (40) is designed to bring about an axial relative movement between the shaft sleeve (31) and the thrust rod (32),
a joint device (20) which is fixedly connected to the plate (10) and can be detachably connected to the shaft (30), wherein the actuating mechanism (40) is adapted to selectively block and release the joint connection (20) by way of the axial relative movement between the shaft sleeve (31) and the thrust rod (32), wherein
the joint connection (20) has a first component (21), which is rigidly attached to the plate (10), and a second component (22), which can be moved and in particular pivoted with respect to the plate (10),
the joint connection (20) is a ball joint connection (21, 22) which has a joint ball (21) and a ball sleeve (22), wherein
the joint ball (21) is provided to be rigid on the plate (10) and the ball sleeve (22) has a thread (23),
the shaft sleeve (30) has a thread (33) and can be screwed to the thread (23) of the ball sleeve (22), and wherein
the thrust rod (32) is adapted to exert a pressure on the joint ball (21) in the actuated state of the actuating device (40) in order to press the joint ball (21) against a seat (24), which is formed on the joint sleeve (22), in order to block the joint connection (20).

2. Atrium retractor according to Claim 1, wherein
the actuating device is adapted to block the relative movement between the shaft sleeve (31) and the thrust rod (32) selectively by means of frictional engagement.

3. Atrium retractor according to Claim 1 or 2, wherein,
in the joint sleeve (22), a support (25) is provided on which an elastic element (26) is supported, which elastic element, on the opposite side, is supported on the joint ball (21) in order to press the joint ball (21) with a predetermined force against the seat (24) in the released state of the joint connection (20) in order to prevent an uncontrolled movement of the joint sleeve (22) with respect to the plate (10), wherein the elastic element (26) is preferably a spring and in particular a leaf spring or plate spring.

4. Atrium retractor according to one of the preceding claims, wherein
the joint sleeve (22) has two parts (22a, 22b) which are detachably or non-detachably connected to one another, wherein the seat (24) and the support (25) are preferably provided on different parts (22a, 22b) of the joint sleeve (22).

5. Atrium retractor according to one of the preceding claims, wherein
the plate (10) is curved and/or has a laterally running projection (13) at its front end (11), said projection protruding from the plate (10) in the ventral direction during use of the atrium retractor.

6. Atrium retractor according to one of the preceding claims, wherein
the plate (10) has a grip region (14) which is adapted to be gripped by an instrument, wherein the grip region (14) is preferably formed such that a pivoting of the plate (10) with respect to the instrument is prevented.

7. Atrium retractor according to one of the preceding claims, with
at least one expansion plate (50) which can be detachably attached to the plate (10) of the atrium retractor, wherein,
in the region of at least one lateral edge, the plate (10) has a groove (15), into which a tongue (51) of the expansion plate (50) can be inserted such that the plate (10) and the expansion plate (50) substantially form a common plate surface.

8. Atrium retractor according to Claim 7, wherein
the at least one groove (15) is formed in the lateral side surface of the plate (10).

9. Atrium retractor according to either of Claims 7 and 8, wherein
the expansion plate (50) is adapted to be plastically flexible in the lateral direction, wherein the expansion plate (50) preferably has at least one cutout (52) which extends from the rear edge to the front region (54) of the expansion plate (50).

10. Atrium retractor according to one of the preceding claims, wherein
the actuating device (40) is an eccentric lever (41) with an eccentric surface (42), wherein the eccentric lever (41) is fastened to the shaft sleeve (31) in an articulated manner and bears, with its eccentric surface (42), on the proximal end of the thrust rod (32).

11. Atrium retractor according to Claim 10, wherein
the eccentric lever (41) is fastened to the shaft sleeve (31) via a grip sleeve (34),
the grip sleeve (34) has a passage bore (35), and the thrust rod (32) has a slot (36), wherein a pin (43) extends through the passage bore (35) in the grip sleeve (34) and the slot (36) in the thrust rod (32) and, in this way, to delimit a maximum movement of the thrust rod (32) with respect to the shaft sleeve (31), in particular in the unactuated state of the actuating device (40).

12. Atrium retractor according to either of Claims 10 and 11, wherein
the eccentric lever (41) has an actuating arm (44) which, in the actuated state of the actuating device (40), runs substantially parallel to the shaft sleeve (31) and preferably substantially bears on the shaft sleeve (31).

13. Atrium retractor according to one of Claims 10 to 12, wherein
the grip sleeve (35) has a projection (37) which engages into a groove (45), said groove being provided in the eccentric lever (41) and extending in the shape of a circular arc about the centre of rotation (46) of the eccentric lever (41), in order to delimit the rotational movement of the eccentric lever (41) in relation to the shaft sleeve (31).

## Revendications

1. Rétracteur atrial, comprenant
une plaque (10) et
un axe (30) qui comporte une gaine d'axe (31), une tige de pression (32) et un mécanisme d'actionnement (40), le mécanisme d'actionnement (40) étant configuré pour provoquer un mouvement relatif axial entre la gaine d'axe (31) et la tige de pression (32),
un dispositif d'articulation (20) qui est relié fixement à la plaque (10) et qui peut être relié de manière amovible à l'axe (30), le mécanisme d'actionnement (40) étant adapté de manière à bloquer et libérer, de manière sélective, la liaison articulée (20) par le biais du mouvement relatif entre la gaine d'axe (31) et la tige de pression (32),
la liaison articulée (20) présentant un premier composant (21) qui est monté rigidement sur la plaque (10) et un deuxième composant (22) qui peut être déplacé et en particulier pivoté par rapport à la plaque (10),
la liaison articulée (20) étant une liaison à articulation à rotule (21, 22) qui présente une rotule d'articulation (21) et une douille à rotule (22),
la rotule d'articulation (21) étant prévue rigidement sur la plaque (10) et la douille à rotule (22) présentant un filetage (23),
la gaine d'axe (30) présentant un filetage (33) et pouvant être vissée avec le filetage (23) de la douille à rotule (22), et
la tige de pression (32) étant adaptée de manière à exercer une pression sur la rotule d'articulation (21), dans l'état actionné du dispositif d'actionnement (40), afin de presser la rotule d'articulation (21) contre un siège (24) qui est réalisé au niveau de la douille d'articulation (22) afin de bloquer la liaison articulée (20) .

2. Rétracteur atrial selon la revendication 1, dans lequel
le dispositif d'actionnement est adapté de manière à bloquer sélectivement, par engagement par friction, le mouvement relatif entre la gaine d'axe (31) et la tige de pression (32).

3. Rétracteur atrial selon la revendication 1 ou 2, dans lequel,
dans la douille d'articulation (22), est prévu un support (25) contre lequel s'appuie un élément élastique (26) qui s'appuie sur le côté opposé contre la rotule d'articulation (21), afin de presser la rotule d'articulation (21), dans l'état libéré de la liaison articulée (20), avec une force prédéterminée contre le siège (24), afin d'empêcher un mouvement non contrôlé de la douille d'articulation (22) par rapport à la plaque (10), l'élément élastique (26) étant de préférence un ressort et en particulier un ressort à lame ou un ressort à coupelle.

4. Rétracteur atrial selon l'une quelconque des revendications précédentes, dans lequel la douille d'articulation (22) présente deux parties (22a, 22b) qui sont connectées l'une à l'autre de manière desserrable ou non desserrable, le siège (24) et le support (25) étant de préférence prévus au niveau de parties différentes (22a, 22b) de la douille d'articulation (22).

5. Rétracteur atrial selon l'une quelconque des revendications précédentes, dans lequel la plaque (10) est cintrée et/ou présente au niveau de son extrémité avant (11) une saillie s'étendant latéralement (13) qui, lors de l'utilisation du rétracteur atrial, fait saillie depuis la plaque (10) dans la direction ventrale.

6. Rétracteur atrial selon l'une quelconque des revendications précédentes, dans lequel la plaque (10) présente une région de préhension (14) qui est adaptée à être prise par un instrument, la région de préhension (14) étant réalisée de préférence de manière à ce qu'un pivotement de la plaque (10) par rapport à l'instrument soit empêché.

7. Rétracteur atrial selon l'une quelconque des revendications précédentes, comprenant au moins une plaque d'élargissement (50) qui peut être montée de manière amovible sur la plaque (10) du rétracteur atrial,
la plaque (10) présentant dans la région d'au moins un bord latéral une rainure (15) dans laquelle peut être enfoncé un ressort (51) de la plaque d'élargissement (50), de telle sorte que la plaque (10) et la plaque d'élargissement (50) forment essentiellement une surface de plaque commune.

8. Rétracteur atrial selon la revendication 7, dans lequel l'au moins une rainure (15) est réalisée dans la surface latérale de la plaque (10).

9. Rétracteur atrial selon l'une quelconque des revendications 7 et 8, dans lequel la plaque d'élargissement (50) est adaptée de manière à pouvoir être fléchie élastiquement dans la direction latérale, la plaque d'élargissement (50) présentant de préférence au moins un évidement (52) qui s'étend depuis le bord arrière jusqu'à la région avant (54) de la plaque d'élargissement (50) .

10. Rétracteur atrial selon l'une quelconque des revendications précédentes, dans lequel le dispositif d'actionnement (40) est un levier excentrique (41) avec une surface excentrique (42), le levier excentrique (41) étant fixé de manière articulée à la gaine d'axe (31) et s'appliquant avec sa surface excentrique (42) contre l'extrémité proximale de la tige de pression (32).

11. Rétracteur atrial selon la revendication 10, dans lequel le levier excentrique (41) est fixé par le biais d'une gaine de préhension (34) à la gaine d'axe (31),
la gaine de préhension (34) présente un alésage traversant (35), et
la tige de pression (32) présente un trou oblong (36), une goupille (43) s'étendant à travers l'alésage traversant (35) dans la gaine de préhension (34) et à travers le trou oblong (36) dans la tige de pression (32) et de cette manière, limiter un mouvement maximal de la tige de pression (32) par rapport à la gaine d'axe (31) en particulier dans l'état non actionné du dispositif d'actionnement (40).

12. Rétracteur atrial selon l'une quelconque des revendications 10 et 11, le levier excentrique (41) présentant un bras d'actionnement (44) qui, dans l'état actionné du dispositif d'actionnement (40), s'étend essentiellement parallèlement à la gaine d'axe (31) et s'applique de préférence essentiellement contre la gaine d'axe (31).

13. Rétracteur atrial selon l'une quelconque des revendications 10 à 12, dans lequel la gaine de préhension (35) dispose d'une saillie (37) qui s'engage dans une rainure (45) qui est prévue dans le levier excentrique (41) et qui s'étend en forme d'arc de cercle autour du centre de rotation (46) du levier excentrique (41) afin de limiter le mouvement de rotation du levier excentrique (41) par rapport à la gaine d'axe (31).
